(19) Europäisches Patentamt
European Patent Office
Office européen des brevets

(11) Veröffentlichungsnummer: **0 157 731**
**B1**

(12) **EUROPÄISCHE PATENTSCHRIFT**

(45) Veröffentlichungstag der Patentschrift: **12.10.88**

(51) Int. Cl.⁴: **C 07 F 9/38,** C 07 C 87/02, C 07 C 149/24, C 10 M 137/12

(21) Anmeldenummer: **85810066.2**

(22) Anmeldetag: **19.02.85**

# (54) Neue Zusätze für Schmierstoffe.

(30) Priorität: **24.02.84 CH 911/84**

(43) Veröffentlichungstag der Anmeldung: **09.10.85 Patentblatt 85/41**

(45) Bekanntmachung des Hinweises auf die Patenterteilung: **12.10.88 Patentblatt 88/41**

(84) Benannte Vertragsstaaten: **BE DE FR GB IT**

(56) Entgegenhaltungen:
**EP-A-0 012 106**
**FR-A-2 132 008**
**US-A-2 858 332**

**Römpps Chemie-Lexikon, Seite 2402 (1974)**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Wirth, Hermann Otto, Dr., Lessingstrasse 24, D-6140 Bensheim 3 (DE)**
Erfinder: **Müller, Klaus, Dr., Am Stadtgraben 7, D-7850 Lörrach (DE)**

**EP 0 157 731 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Ammoniumsalze der Methylphosphonsäure und deren Verwendung als Zusätze für Schmierstoffe, sowie Zusammensetzungen enthaltend die neuen Verbindungen und ein Verfahren zur Schmierung von Maschinenteilen.

Aus EP-A-12 106 sind Ammoniumsalze der Methylphosphonsäure als Verschleiss- und Hochdruckwirkstoffe für wassermischbare Hydraulikflüssigkeiten bekannt. Das Ammoniumkation dieser Verbindungen enthält mindestens eine β-Hydroxyethylgruppe, was den Salzen eine Löslichkeit in polaren Flüssigkeiten verleiht.

α-Chlormethylphosphonsäuren in Kombination mit primären Alkylaminen, deren Alkylrest mehr als acht C-Atome enthält und der bevorzugt verzweigt ist, werden in der US-PS-2 858 332 als Hochdruckwirkstoffe beschrieben. Je nach Verwendungszweck wird dort das Monoamino-oder das Diaminoderivat bevorzugt eingesetzt.

Aus der FR-PS-2 132 008 sind weiters Aminsalze von phosphorhaltigen Säuren der Reihe der Phosphate, der Phosphonate und der Phosphinate bekannt geworden. Das anzuwendende Amin ist ein Phenylakylamin und die Verbindungen sollen in Ölmischung besonders mit elastomeren Verbindungen verträglich sein.

Es ist jetzt überraschenderweise gefunden worden, dass Salze der Methylphosphonsäure mit primären Aminen, insbesondere Salze, die weniger als zwei Äquivalente an Amin enthalten, Schmierstoffen ungewöhnlich gute Hochdruckeigenschaften verleihen. Diese Verbindungen übertreffen darin sogar die als sehr wirksam bekannten Thiaphosphetan-Derivate.

Die vorliegende Erfindung betrifft Verbindungen der Formel I

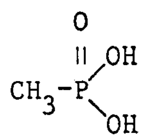 • $[R-NH_2]_n$ (I)

worin n eine Zahl zwischen 1,0 und 2,2 bedeutet und worin R geradkettiges oder verzweigtes $C_8$-$C_{30}$ Alkyl bedeutet, das gegebenenfalls ein- bis fünfmal durch -S- unterbrochen, oder worin R geradkettiges oder verzweigtes $C_8$-$C_{30}$ Alkenyl ist.

R bedeutet als geradkettiges $C_8$-$C_{30}$ Alkyl n-Octyl, n-Decyl, n-Undecyl, n-Dodecyl, n-Tridecyl, n-Tetradecyl, n-Hexadecyl, n-Octadecyl, n-Eicosyl, n-Docosyl oder n-Triacontyl. Als verzweigtes $C_8$-$C_{30}$ Alkyl bedeutet R beispielsweise 1,1,3,3-Tetramethylbutyl, 1,1,3,3,5,5-Hexamethylhexyl, 1,1,3,3,5,5,7,7-Octamethyloctyl oder 1,1,3,3,5,5,7,7,9,9-Decamethyldecyl.

Bedeutet R $C_8$-$C_{30}$ Alkenyl, so kommt insbesondere Oleyl in Frage. Bevorzugtverwendet man primäre Aminemit einen verzweigten $C_8$-$C_3$ Alkylrest R, insbesondere solche, die tert.-C-Atome enthalten, und besonders bevorzugt solche, mit einem tert.-C-Atom in α-Stellung zur Aminogruppe. Diese Amine können beispielsweise aus verzweigten primären Alkoholen, die im Oxo-Prozess anfallen, nach gängigen Methoden erhalten werden. Solche Umsetzungen werden von P.W. Sherwood im "Oil and Gas Journal, 1949, 71 beschrieben. Vorzugsweise verwendet man Mischungen solcher Amine, wie sie im Handel unter Bezeichnung "Primene" erhältlich sind. So lässt sich beispielsweise die Mischung "Primene® 81-R" (hauptsächlich verzweigte Alkylamine mit 12 bis 15 C-Atome) oder die Mischung "Primene® JM-T" (hauptsächlich verzweigte primäre Alkylamine mit 18 bis 24 C-Atomen) einsetzen.

Weiterhin bevorzugt wird R als durch ein bis fünf Schwefelatome unterbrochenes $C_8$-$C_{30}$ Alkyl; insbesondere als durch ein bis fünf Schwefelatome unterbrochenes verzweigtes $C_8$-$C_{30}$ Alkyl; besonders bevorzugt wird R als $-CH_2CH_2$-S-Alkyl ($C_9$-$C_{12}$), insbesondere $-CH_2-CH_2$-S-tert.-$C_{12}H_{25}$ und $-CH_2-CH_2$-S-tert.-$C_9$ $H_{19}$. Dabei ist unter tert.-$C_9$-$H_{19}$ bzw. tert.-$C_{12}H_{25}$ ein Gemisch verschiedener Isomere zu verstehen, die vorzugsweise dadurch gekennzeichnet sind, dass sich in α-Stellung ein tert.-C-Atom befindet. Selbstverständlich kann der $C_9$-$C_{12}$ Alkylrest auch ein Gemisch verzweigter Alkylreste mit unterschiedlicher Kohlenstoffzahl darstellen.

Ebenfalls bevorzugt werden Verbindungen der Formel I, worin n 1,0 bis 1,5 bedeutet, oder worin R $C_{10}$-$C_{30}$ Alkyl, insbesondere verzweigtes Alkyl, welches in α-Stellung zur Amino Gruppe ein tert.-C-Atom besitzt, bedeutet.

Die Herstellung der Verbindungen der Formel (I) kann auf an sich bekannte Weise erfolgen. Am einfachsten wird Methylphosphonsäure der Formel II

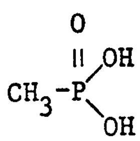 (II)

mit einem Amin der Formel III

$$R-NH_2 \quad (III),$$

worin R die oben erwähnte Bedeutung hat, umgesetzt. Das Mol-Verhältnis beträgt insbesondere 1 : 1 bis 1 : 2,2, bevorzugt 1 : 1,5. Gegebenenfalls verwendet man ein Lösungs- bzw. Verdünnungsmittel, wie einen Alkohol, beispielsweise Methanol, der nach erfolgter Umsetzung z. B. unter reduziertem Druck abdestilliert werden

kann. Man erhält das Aminsalz als ein viskoses Öl.

Die verwendeten Ausgangsprodukte sind bekannte Verbindungen, die auf einfache Weise herstellbar sind.

Die Verbindungen der Formel I werden in Konzentrationen von 0,01 bis 10 Gew.-%, bezogen auf das Gesamtgewicht der Schmierstoff-Formulierung, eingesetzt. Bevorzugter Konzentrationsbereich ist 0,05 bis 5,0 Gew.-%.

Die erfindungsgemässen Verbindungen sind aufgrund ihrer das Hochdruckverhalten verbessernden und Korrosionsschutz vermittelnden Eigenschaften als Zusätze für synthetische Schmieröle oder Schmierfette sowie für Schmierstoffe auf Mineralölbasis geeignet. Auch lassen sich die Verbindungen der Formel 1 in Metallbearbeitungsölen einsetzen. Die Erfindung betrifft daher auch Schmierstoffe enthaltend Verbindungen der Formel I, sowie die Verwendung von Verbindungen der Formel I als Hochdruck- und Verschleisszusätze in Schmierstoffen. Ferner betrifft die Erfindung ein Verfahren zur Schmierung von Maschinenteilen mit starker Schockbelastung unter Verwendung eines Schmierstoffes enthaltend eine Verbindung der Formel I.

Die in Frage kommenden Schmierstoffe sind dem Fachmann geläufig und z. B. im "Schmiermittel-Taschenbuch (Hüthig Verlag, Heidelberg, 1974)" beschrieben. Besonders geeignet sind neben Mineralölen z. B. Poly-α-Olefine, Schmierstoffe auf Carbonsäureesterbasis, Phosphatester, Polyalkylenglykole und Mischungen der genannten Schmierstoffe.

Die Schmierstoff-Formulierungen können zusätzlich noch andere Additive enthalten, die zugegeben werden, um gewisse Gebrauchs-Eigenschaften zu verbessern, wie z. B. weitere Antioxidantien, Metallpassivatoren, Rostinhibitoren, Viskositätsindex-Verbesserer, Stockpunkterniedriger, Dispergiermittel/Tenside und Verschleißschutz-Additive.

**Beispiele für phenolische Antioxidantien**

1. Alkylierte Monophenole
2,6-Di-tert.-butylphenol
2-tert.-butyl-4,6-Dimethylphenyl
2,6-Di-tert.-butyl-4-ethylphenol
2,6-Di-tert.-butyl-4-ethylphenol
2,6-Di-tert.-butyl-4-n-butylphenol
2,6-Di-tert.-butyl-4-i.-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.-butyl-4-methoxymethylphenol

2. Alkylierte Hydrochinone
2,6-Di-tert.-butyl-4-methoxyphenol
2,5-Di-tert.-butylhydrochinon
2,5-Di-tert.-amylhydrochinon
2,6-Diphenyl-4-octadecyloxyphenol

3. Hydroxylierte Thiodiphenylether
2,2'-Thio-bis-(6-tert.-butyl-4-methylphenol)
2,2'-Thio-bis-(4-octylphenol)
4,4'-Thio-bis-(6-tert.-butyl-3-methylphenol)
4,4'-Thio-bis-(6-tert.-butyl-2-methylphenol)

4. Alkyliden-Bisphenole
2,2'-Methylen-bis-(6-tert.-butyl-4-methylphenol)
2,2'-Methylen-bis-(6-tert.-butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.-butylphenol)
2,2'-Ethyliden-bis-(4,6-di-tert.-butylphenol)
2,2'-Ethyliden-bis-(6-tert.-butyl-4-isobutyphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(6-tert.-butyl-2-methylphenol)
1,1-Bis-(5-tert.-butyl-4-hydroxy-2-methylphenyl)-butan
2,6-Di-(3-tert.-butyl-5-methyl-2-hydroxybenzyl)-4-methylphenol
1,1,3-Tris-(5'-tert.-butyl-4'-hydroxy-2'-methylphenyl)-3-n-dodecylmercaptobutan

Ethylenglycol-bis-[3,3-bis-(3'-tert.-butyl-4'-hydroxyphenyl)-butyrat]
Di-(3-tert.-butyl-4-hydroxy-5-methylphenyl)-dicyclopentadien
Di-[2-(3'-tert.-butyl-2'-hydroxy-5'-methylbenzyl)-6-tert.-butyl-4methyl-phenyl]-terephthalat.

5. Benzylverbindungen
1,3,5-Tri-(3'-5'-di-tert.-butyl-4'-hydroxybenzyl)-2,4,6-trimethylbenzol
Di-(3,5-di-tert.-butyl-4-hydroxybenzyl)-sulfid
Bis-(4-tert.-butyl-3-hydroxy-2,6-dimethylbenzyl)dithiolterephthalat
1,3,5-Tris-(3',5'-di-tert.-butyl-4'-hydroxybenzyl)-isocyanurat
1,3,5-Tris-(4'-tert.-butyl-3'-hydroxy-2',6'-dimethylbenzyl)-isocyanurat
3,5-Di-tert.-butyl-4-hydroxybenzylphosphonsäure-dioctadecylester
3,5-Di-tert.-butyl-4-hydroxybenzylphosphonsäuremonoethylester Calcium-salz.

6. Acylaminophenole
4-Hydroxy-laurinsäureanilid
4-Hydroxy-stearinsäureanilid
2,4-Bis-octylmercapto-6-(3',5'-di-tert.-butyl-4'-hydroxyanilino)-s-triazin
N-(3,5-di-tert.-butyl-4-hydroxyphenyl)-carbaminsäureoctylester.

7. Ester der β-(3,5-Di-tert.-butyl-4-hydroxyphenyl)-propionsäure
mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethylisocyanurat1 |
| Thiodiethylenglycol | Di-hydroxyethyloxalsäurediamid |

8. Ester der β-(5-tert.-butyl-4-hydroxy-3-methylphenyl)-propionsäure
mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethylisocyanurat |
| Thiodiethylenglycol | Di-hydroxyethyloxalsäurediamid |

9. Amide der β-(3,5-Di-tert.-butyl-4-hydroxyphenyl)-propionsäure,
wie z. B.
N,N'-Bis-(3,5-di-tert.-butyl-4-hydroxyphenylpropionyl)-hexamethylendiamin
N,N'-Bis-(3,5-di-tert.-butyl-4-hydroxyphenylpropionyl)-trimethylendiamin
N,N'-Bis-(3,5-di-tert.-butyl-4-hydroxyphenylpropionyl)-hydrazin.

**Beispiele für aminische Antioxidantien:**

N,N'-Di-isopropyl-p-phenylendiamin
N,N'-Di-sec.-butyl-p-phenylendiamin
N,N'-Bis-(1,4-dimethylpentyl)-p-phenylendiamin
N,N'-Bis-(1-ethyl-3-methylpentyl)-p-phenylendiamin
N,N'-Bis-(1-methylheptyl)-p-phenylendiamin
N,N'-Dicyclohexyl-p-phenylendiamin
N,N'-Diphenyl-p-phenylendiamin
N,N'-Di-(naphthyl-2-)-p-phenylendiamin
N-Isopropyl-N'-phenyl-p-phenylendiamin
N-(1,3-Dimethylbutyl)-N'-phenyl-p-phenylendiamin

N-(1-Methylheptyl)-N'-phenyl-p-phenylendiamin
N-Cyclohexyl-N'-phenyl-p-phenylendiamin
Bis-4-(p-Toluol-sulfonamidophenyl)-amin
N,N'-Dimethyl-N,N'-di-sec.-butyl-p-phenylendiamin Diphenylamin
4-Isopropoxydiphenylamin
N-Phenyl-1-naphthylamin
N-Phenyl-2-naphthylamin
octyliertes Diphenylamin
4-n-Butylaminophenol
4-Butyrylaminophenol
4-Nonanoylaminophenol
4-Dodecanoylaminophenol
4-Octadecanoylaminophenol
Bis-(4-methoxyphenyl)-amin
2,6-Di-tert.-butyl-4-dimethylaminomethylphenol
2,4'-Diaminodiphenylmethan
4,4'-Diaminodiphenylmethan
N,N,N',N'-Tetramethyl-4,4'-diaminodiphenylmethan
1,2-Bis-(phenylamino)-ethan
1,2-Bis-[(2-methylphenyl)-amino]-ethan
1,3-Bis-(phenylamino)-propan
(o-Tolyl)-biguanid
Bis-[4-(1',3'-dimethylbutyl)-phenyl]-amin

**Beispiele für Metallpassivatoren:**

für Kupfer, z. B.:
Benztriazol, Tetrahydrobenztriazol, 2-Mercaptobenzthiazol 2,5-Dimercaptothiadiazol, Salicylidenpropylendiamin, Salze von Salicylaminoguanidin.

**Beispiele für Rost-Inhibitoren:**

a) Organische Säuren, ihre Ester, Metallsalze und Anhydride, z. B.: N-Oleoylsarcosin, Sorbitan-monooleat Bleinaphthenat, Dodecenylbernsteinsäureanhydrid, Alkenylbernsteinsäure-Halbester, 4-Nonylphenoxyessigsäure.
b) Stickstoffhaltige Verbindungen, z. B.:
I. Primäre, sekundäre oder tertiäre aliphatische oder cycloaliphatische Amine und Amin-Salze von organischen und anorganischen Säuren, z. B. öllösliche Alkylammoniumcarboxylate.
II. Heterocyclische Verbindungen z. B.:
Substituierte Imidazoline und Oxazoline.
c) Phosphorhaltige Verbindungen, z. B.:
Aminsalze von Phosphorsäurepartialestern.
d) Schwefelhaltige Verbindungen, z. B.:
Bariumdinonylnaphthalinsulfonate, Calciumpetroleumsulfonate.

**Beispiele für Viskositätsindex-Verbesserer:**

Polymethacrylate, Vinylpyrrolidon/Methacrylat-Copolymere, Polybutene, Olefin-Copolymere, Styrol/Acrylat-Copolymere.

**Beispiele für Stockpunkterniedriger:**

Polymethacrylat, alkylierte Naphthalinderivate.

**Beispiele für Dispergiermittel/Tenside:**

Polybutenylbernsteinsäureimide, Polybutenylphosphonsäurederivate, basische Magnesium-, Calcium-, und Bariumsulfonate und -phenolate.

**Beispiele für Verschleißschutz-Additive:**

Schwefel und/oder Phosphor und/oder Halogen enthaltende Verbindungen, wie geschwefelte pflanzliche Öle Zinkdialkyldithiophosphate, Tritolylphosphat, chlorierte Paraffine, Alkyl- und Aryldisulfide.

**Herstellungsbeispiele 1 - 4:**

Die in der folgenden Tabelle angegebenen Salze werden durch Zusammengeben entsprechender molarer Anteile von Methylphosphonsäure und primärem Amin erhalten.

<u>Tabelle</u>

| Substanz Nr. | | Formel | Eigenschaften |
|---|---|---|---|
| 1 | FIG1/4 | · [iso-$C_{13}H_{27}NH_2$] 1) | gelbe viskose Flüssigkeit $n^{20}_D = 1{,}4612$ |
| 2 | FIG2/4 | · [iso-$C_{13}H_{27}NH_2$] 1) + 25 % Paraffinöl | gelbe viskose Flüssigkeit |
| 3 | FIG3/4 | · [iso-$C_{13}H_{27}NH_2$]$_{1,5}$ 1) | gelbe viskose Flüssigkeit |
| 4 | FIG4/4 | · [iso-$C_{20}H_{41}NH_2$]$_{1,5}$ 2) | gelbe viskose Flüssigkeit |

1) PRIMENE 81-R®
2) PRIMENE JM-T®

**Beispiel 5:**

Mit dem Shell-Vierkugel-Apparat (IP 239/73 Extreme pressure and wear lubricant test for oils and greases four ballmachine) wird der W.L.-Wert und die Verschleisskalotte (WSD) in Abhängigkeit der Konzentration des Hochdruckzusatzes bestimmt.

W.L. = Weld load (Schweisslast). Das ist die Last, bei der die 4 Kugeln innerhalb von 10 Sekunden zusammenschweissen.

WSD = Wear Scar Diameter (Verschleisskalotte). Das ist der mittlere Durchmesser der Verschleisskalotten auf den 3 Standkugeln nach 10 Minuten bei 400 N Last.

Als Testflüssigkeit für die Wirksamkeit der Additive wird Mineralöl der Viskositätsklasse ISO VG 100 verwendet. Die Ergebnisse sind in der nachfolgenden Tabelle aufgeführt:

<u>Tabelle:</u>

| Additiv (Produkt Nr.) | Konzentration (Gew.-%) | W.L. (N) | WSD nach 10 Min. und 400 N (mm) |
|---|---|---|---|
| - | - | 1450 | - |
| 1 | 1,0 | 3750 | 0,50 |
| 1 | 2,0 | 6500 | 0,53 |
| 1 | 5,0 | 9500 | - |
| 1 | 10,0 | 9500 | - |

**Patentansprüche**

1. Verbindungen der Formel I

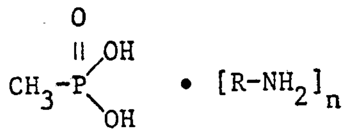

(I) ,

worin n eine Zahl zwischen 1,0 und 2,2 ist und R geradkettiges oder verzweigtes $C_8$-$C_{30}$ Alkyl bedeutet, das gegebenenfalls durch ein bis fünf Schwefelatome unterbrochen ist, oder worin R geradkettiges oder verzweigtes $C_8$-$C_{30}$ Alkenyl ist.

2. Verbindungen der Formel I gemäss Anspruch 1, worin n 1,0 bis 1,5 ist.

3. Verbindungen der Formel I gemäss Anspruch 1, worin R verzweigtes $C_8$-$C_{30}$ Alkyl bedeutet.

4. Verbindungen der Formel I gemäss Anspruch 1, worin R verzweigtes durch ein bis fünf Schwefelatome unterbrochenes $C_8$ -$C_{30}$ Alkyl bedeutet.

5. Verbindungen der Formel I gemäss Anspruch 1, worin R $C_{10}$ -$C_{30}$ Alkyl bedeutet und in α-Stellung zur Aminogruppe ein tert.-C-Atom besitzt.

6. Eine Verbindung der Formel IV gemäss Anspruch 1

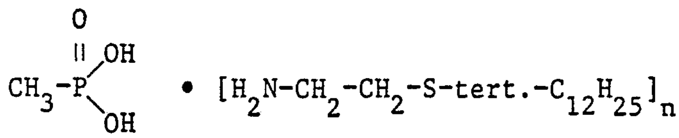

(IV) ,

worin n die Zahl 1 ist.

7. Eine Verbindung der Formel V gemäss Anspruch 1

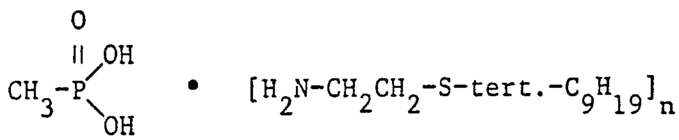

(V),

worin n die Zahl 1 ist.

8. Verbindung der Formel I gemäss Anspruch 1, worin als Aminkomponente ein Gemisch von verzweigten $C_{12}$-$C_{15}$ Alkylaminen, welche in α-Stellung ein tert. C-Atom enthalten, verwendet wird.

9. Verbindung der Formel I gemäss Anspruch 1, worin als Aminkomponente ein Gemisch von verzweigten $C_{18}$-$C_{24}$ Alkylaminen, welche in α-Stellung ein tert. C-Atom enthalten, verwendet wird.

10. Schmierstoffe enthaltend Verbindungen der Formel I gemäss Anspruch 1 als Hochdruck- und Verschleisszusätze.

11. Verwendung von Verbindungen der Formel I gemäss Anspruch 1 als Hochdruck- und Verschleisszusätze in Schmierstoffen.

12. Verfahren zur Schmierung von Maschinenteilen mit starker Schockbelastung, dadurch gekennzeichnet, dass man dem Schmierstoff eine Verbindung der Formel I gemäss Anspruch 1 oder eine Mischung solcher Verbindungen zusetzt.

**Claims**

1. A compound of the formula I

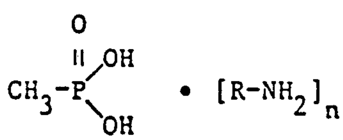

(I)

wherein n is a number from 1.0 to 2.2 and R is straight chain or branched $C_8$-$C_{30}$alkyl which may be interrupted by one to five sulfur atoms, or wherein R is straight chain or branched $C_8$-$C_{30}$alkenyl.

2. A compound of formula I according to claim 1, wherein n is 1.0 to 1.5.

3. A compound of formula I according to claim 1, wherein R is branched $C_8$-$C_{30}$alkyl.

4. A compound of formula I according to claim 1, wherein R is branched $C_8$-$C_{30}$alkyl which is interrupted by one to five sulfur atoms.

5. A compound of formula I according to claim 1, wherein R is $C_{10}$-$C_{30}$alkyl and has a tertiary carbon atom in the α-position relative to the amino group.

6. A compound of formula IV according to claim 1

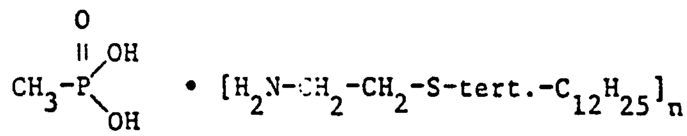 (IV)

wherein n is 1.

7. A compound of formula V according to claim 1

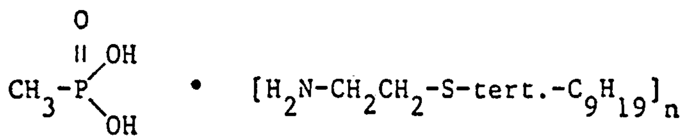 (V)

wherein n is 1.

8. A compound of formula I according to claim 1, wherein the amine component used is a mixture of branched $C_{12}$-$C_{15}$alkylamines which contain a tertiary carbon atom in the α-position.

9. A compound of formula I according to claim 1, wherein the amine component used is a mixture of branched $C_{18}$-$C_{24}$alkylamines which contain a tertiary carbon atom in the α-position.

10. A lubricant containing a compound of formula I according to claim 1 as extreme pressure and antiwear additives.

11. The use of a compound of formula I according to claim 1 as extreme pressure and antiwear additives in lubricants.

12. A process for lubricating machine parts which are subject to severe shock stress, which process comprises adding a compound of formula I according to claim 1 or a mixture of such compounds to the lubricant.

**Revendications**

1. Composés répondant à la formule I:

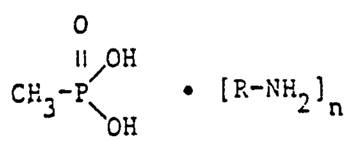 (I)

dans laquelle n représente un nombre compris entre 1,0 et 2,2, et R représente un radical alkyle en $C_8$-$C_{30}$ linéaire ou ramifié, qui est éventuellement interrompu par un à cinq atomes de soufre ou R représente un radical alcényle en $C_8$-$C_{30}$, linéaire ou ramifié.

2. Composés de formule I selon la revendication 1 dans lesquels n désigne un nombre compris entre 1,0 et 1,5.

3. Composés de formule I selon la revendication 1 dans lesquels R représente un radical alkyle $C_8$-$C_{30}$ ramifié.

4. Composés de formule I selon la revendication 1 dans lesquels R représente un radical alkyle en $C_8$-$C_{30}$ ramifié et interrompu par un à cinq atomes de soufre.

5. Composés de formule I selon la revendication 1 dans lesquels R représente un radical alkyle en $C_8$-$C_{30}$ et contient un atome de carbone tertiaire en position α par rapport au radical amino.

6. Composé selon la revendication 1, en l'espèce celui qui répond à la formule IV:

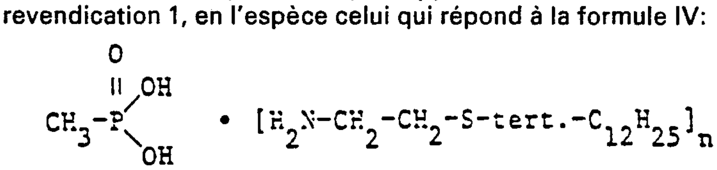 (IV)

dans laquelle n désigne le nombre 1.

7. Composé selon la revendication 1, en l'espèce celui qui répond à la formule V:

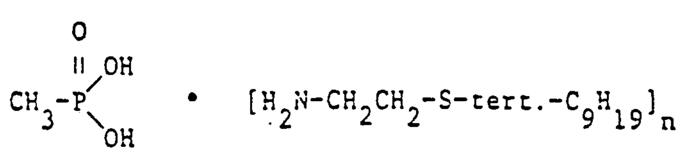 (V)

dans laquelle n désigne le nombre 1.

8. Composé de formule I selon la revendication 1 dans lequel la composante amine utilisée est un mélange d'alkylamines en $C_{12}$-$C_{15}$ ramifiées qui contiennent un atome de carbone tertiaire en position $\alpha$.

9. Composé de formule I selon la revendication 1 dans lequel la composante amine utilisée est un mélange d'alkylamines en $C_{18}$-$C_{24}$ ramifiées qui contiennent un atome de carbone tertiaire en position $\alpha$.

10. Lubrifiants contenant des composés de formule I selon la revendication 1 comme additifs anti-usures et haute pression.

11. Application de composés de formule I selon la revendication 1 comme additifs anti-usures et haute pression dans des lubrifiants.

12. Procédé pour lubrifier des pièces de machines soumises à une forte charge par à-coups, procédé caractérisé en ce qu'on ajoute au lubrifiant un composé de formule I selon la revendication 1 ou un mélange de tels composés.